(19) Europäisches Patentamt
European Patent Office
Office européen des brevets

(11) EP 2 313 082 B1

(12) **EUROPEAN PATENT SPECIFICATION**

(45) Date of publication and mention of the grant of the patent:
**23.08.2017 Bulletin 2017/34**

(21) Application number: **08792967.5**

(22) Date of filing: **31.07.2008**

(51) Int Cl.:
***A61Q 19/08*** (2006.01) ***A61K 8/97*** (2017.01)

(86) International application number:
**PCT/KR2008/004452**

(87) International publication number:
**WO 2010/013852 (04.02.2010 Gazette 2010/05)**

(54) **COSMETIC COMPOSITION FOR ANTI-AGING OF THE SKIN COMPRISING PHASEOLUS RADIATUS SEED EXTRACTS BY FERMENTATION AND ENZYME TREATMENT**

KOSMETISCHE ZUSAMMENSETZUNG GEGEN ALTERUNG DER HAUT MIT EXTRAKTEN AUS DEM SAMEN VON PHASEOLUS RADIATUS DURCH FERMENTATION UND ENZYMBEHANDLUNG

COMPOSITION COSMÉTIQUE POUR L'ANTIVIEILLISSEMENT DE LA PEAU, RENFERMANT DES EXTRAITS DE GRAINE DE PHASEOLUS RADIATUS PAR FERMENTATION ET TRAITEMENT ENZYMATIQUE

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**27.04.2011 Bulletin 2011/17**

(73) Proprietor: **Coreana Cosmetics Co., Ltd.**
**330-833 Cheonan-shi,**
**Chungcheongnam-do (KR)**

(72) Inventors:
- **LEE, Ghang Tai**
**Chungcheongnam-do 330-835 (KR)**
- **LEE, Jung Noh**
**Chungcheongnam-do 339-752 (KR)**
- **YOU, Young Kyoung**
**Chungcheongbuk-do 360-813 (KR)**
- **LEE, Kwang Sik**
**Chungcheongnam-do 330-090 (KR)**
- **LEE, Seung Ji**
**Chungcheongnam-do 330-786 (KR)**
- **LEE, Kun Kook**
**Seoul 138-788 (KR)**

(74) Representative: **Jacobacci Coralis Harle**
**32, rue de l'Arcade**
**75008 Paris (FR)**

(56) References cited:
**WO-A1-2007/007989**
**JP-A- 2007 126 368**
**KR-A- 20040 042 976**
**KR-A- 20070 111 025**
**KR-B1- 100 753 310**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 313 082 B1

## Description

### Technical Field

The present invention relates to a cosmetic composition comprising *Phaseolus radiatus* extract of fermentation-enzyme treatment; and, more particularly, to a cosmetic composition comprising *Phaseolus radiates* extract of fermentation-enzyme treatment as an active ingredient obtained by the secondary enzyme treatment of protease to fermented *Phaseolus radiates* prepared by fermenting *Phaseolus radiatus* with yeast or lactic acid bacterium.

### Background Art

As aging progresses, human skin demonstrates various senile changes. In general, as aging goes on, skin becomes dry, loses elasticity, produces wrinkles and becomes dark. In addition, skin becomes more sensitive to a external stimulus but requires longer time to be recovered. Such senile changes are natural and cannot be 100 % prevented. However, it is possible to postpone aging or make the progress of aging slow by any means. So, most of cosmetics are produced mainly to prevent aging.

One of the most effective ways to inhibit skin aging by using a cosmetic is to add various anti-aging ingredients to a cosmetic. So, cosmetic companies have been trying to develop and screen anti-aging ingredients. Recently, an active ingredient obtained by fermentation has been applied to a cosmetic product.

For example, fermented liquor obtained by using *Bacillus subtillis* which has been used for production of fermented food such as fermented soybeans and natto has been applied to a cosmetic composition. Such fermented liquor is characterized by decomposition or transformation of irritating components by a microorganism, which can alleviate skin irritation. So, fermented product prepared by a microorganism is now believed to have positive effect on skin, so that it is applied to a cosmetic composition.

Moreover, WO-2007/007989 discloses a skin care compositions comprising an extract of *Phaseolus radiates* which has been fermented with lactic acid bacteria; KR-100 753 310 discloses a skin mask containing an extract of fermented *Phaseolus radiatus.*

### Disclosure of the Invention

### Technical Problem

Thus, the present inventors tried to improve various skin troubles caused by aging. As a result, the inventors completed this invention by confirming that fermented *Phaseolus radiatus* extract obtained by the fermentation of *Phaseolus radiatus* using yeast or lactic acid bacterium had excellent anti-aging effect, and particularly confirming that when the fermented *Phaseolus radiatus* extract was treated secondarily with protease, the resultant product could impressively improve skin troubles caused by aging such as dryness and wrinkles.

### Technical Solution

An embodiment of the present invention is directed to providing a cosmetic composition comprising *Phaseolus radiatus* extract offermentation-enzyme treatment for preventing skin aging.

Another embodiment of the present invention is directed to providing a cosmetic composition comprising *Phaseolus radiatus* extract offermentation-enzyme treatment for improving skin wrinkles.

Another embodiment of the present invention is directed to providing a cosmetic composition comprising *Phaseolus radiatus* extract offermentation-enzyme treatment for skin moisturizing.

Another embodiment of the present invention is directed to providing a make-up method including the step of applying the cosmetic composition comprising *Phaseolus radiatus* extract offermentation-enzyme treatment on skin to bring the effect of accelerating collagen biosynthesis and skin cell activity and moisturizing.

### Advantageous Effects

The present invention relates to a cosmetic composition comprising *Phaseolus radiatus* extract of fermentation-enzyme treatment for preventing skin aging. The active ingredient *Phaseolus radiatus* extract of fermentation-enzyme treatment included in the cosmetic composition of the present invention is obtained by the processes of fermenting *Phaseolus radiatus* using yeast or lactic acid bacterium; and secondarily treating the fermented liquor with protease. The extract of the present invention can accelerate cellular activity and collagen synthesis and alleviate skin irritation. So, a cosmetic composition comprising such extract has the effect of improving wrinkles and moisturizing, suggesting that the composition is an excellent candidate for a cosmetic composition that will bring the excellent preventive effect of skin aging.

**Best Mode for Carrying out the Invention**

To achieve the objects of the present invention, the present invention provides a cosmetic composition comprising *Phaseolus radiatus* extract offermentation-enzyme treatment for preventing skin aging.
Further, the present invention provides a cosmetic composition comprising *Phaseolus radiatus* extract offermentation-enzyme treatment for improving skin wrinkles and moisturizing the skin.
*Phaseolus radiatus* (Mung bean) used as the active ingredient in the composition of the present invention is the annual plant belonging to Leguminosae family. This plant grows on loamy soil (black soil in which sand and clay are properly mixed) under moderate climate. The height of the plant is 30 ~ 80 cm. It has thin stalks and vertical veins. There are about 10 gnarls. A pair of seed leaves and new leaves come out first and then three small compound leaves are coming out.
Unlike other Leguminosae family members, *Phaseolus radiatus* has long been used for the treatment of skin disease and as an antipyretic drug and antidote. According to Dongeuibogam (the name of an old Korean book on medicine), when *Phaseolus radiatus* powder was applied on face, cosmetic effect was demonstrated. Up to date, *Phaseolus radiatus* has been used as dried powder or mixed with water or honey for face massage. Or *Phaseolus radiatus* has been eaten as food, for example *Phaseolus radiatus* powder is mixed with water to make *Phaseolus radiatus* pan cake or *Phaseolus radiatus* starch is used for the production of Mung bean starch gel, Mung bean pan cake, Mung bean gruel and green bean sprouts.
In this specification, *Phaseolus radiatus* means all the organs of the *Phaseolus radiatus* plant (including seed (fruit), leaf, flower, stem and root), and more preferably it indicates the seed (fruit).
In this specification, the term *Phaseolus radiatus* extract offermentation-enzyme treatment indicates the extract obtained by the processes of primary fermentation of *Phaseolus radiatus* using fermentative organism to give fermented *Phaseolus radiatus* extract; and secondary enzyme treatment of the extract with protease. According to the kind of fermentative organism, the extract is divided into *Phaseolus radiatus* extract of yeastfermentation-enzyme treatment and *Phaseolus radiatus* extract oflactic acid bacterium fermentation-enzyme treatment. Herein, yeast or lactic acid bacterium is preferably used as the fermentative organism. When yeast is used as the fermentative organism, the extract is understood as *Phaseolus radiatus* extract ofyeast fermentation-enzyme treatment and when lactic acid bacterium is used as the fermentative organism, the extract is understood as *Phaseolus radiatus* extract oflactic acid bacterium fermentation-enzyme treatment.
On the other hand, the term *Phaseolus radiatus* extract ofenzyme treatment-fermentation means the extract obtained by the reverse processes of the above *Phaseolus radiatus* extract offermentation-enzyme treatment production processes, which means the step of fermentation and the step of enzyme treatment are performed in reverse order. That is, *Phaseolus radiatus* extract is first treated with protease and then fermentation is induced using a fermentative organism.
Yeast used for fermenting *Phaseolus radiatus* herein is one of eukaryotic microorganisms (Nucleus and cytoplasm are separated by nuclear membrane.). It has cell organelles such as cell wall, cell membrane, nucleus, mitochondria and granules. This amphigamous organism is propagated by budding. There are various types of yeasts. Some of those separated yeasts have no fermenting ability, some are propagated by fission or bud-fission instead of budding, and some of them form ballistopores instead of ascospores or some do not form spores. The size is 5 ~ 10 $\mu$m $\times$ 5 ~ 12 $\mu$m in average, which is 5 ~ 10 times the size of bacterium. Optimum pH for them is 4.5 ~ 5.5, but they can survive at pH 1 ~ 10. Yeast that can survive about 12 hours at pH 1 is stronger in acid than bacteria. So, contamination by bacteria can be prevented if pH of medium is adjusted to 3.5 ~ 3.8.
Yeast is growing well at 20 ~ 25 °C, even if optimum temperature varies with strains. It is generally known that yeast is killed at 50 °C or higher, which though can be overcome by adding a growth factor that is not synthesized at that temperature or up directly to the medium. *Saccharomyces cerevisiae* is killed at 40 °C or up, but when ergosterol and oleic acid are added to the culture medium, they can survive. Yeast cell itself is a unicellular plant and contains plenty of proteins and minerals. The preferable yeast for the fermentation of *Phaseolus radiatus* herein is *Saccharomyces cerevisiae.*
Lactic acid bacterium used as a fermentative organism for fermenting *Phaseolus radiatus* herein is a kind of beneficial microorganisms that produce organic acid such as lactic acid or acetic acid by decomposing carbohydrate such as glucose or lactose. In general, when lactic acid bacterium produces lactic acid from lactose, it is called fermentation. Fermented food is produced by such fermentation process. Lactic acid bacterium used in this invention is exemplified by *Lactobacillus, Streptococcus, Bifidobacterium, Leuconostoc, Pediococcus, Lactococcus,* etc, and more preferably *Lactobacillus.*
The *Phaseolus radiatus* extract offermentation-enzyme treatment of the present invention is prepared by the following steps:

a) grinding *Phaseolus radiatus* to powder;
b) diluting the *Phaseolus radiatus* powder prepared in step a) with purified water, and then fermenting the diluent using yeast or lactic acid bacterium;
c) filtering the fermented *Phaseolus radiatus* obtained by the fermentation in step b), and then treating the filtrate with protease; and
d) preparing *Phaseolus radiatus* extract offermentation-enzyme treatment by cold-aging and filtering the enzyme treated solution of step c).

In the preparation method of *Phaseolus radiatus* extract offermentation-enzyme treatment, *Phaseolus radiatus* is grinded in step a) into 100 ~ 500 mesh sized or minuter powder, but not always limited thereto.

In step b), fermentation is performed as follows; *Phaseolus radiatus* powder is mixed with purified water at the ratio of 1:10, which is sterilized at 121 °C, followed by inoculation with *Lactobacillus* sp or *Saccharomyces* sp and cultivation.

The preferable amount of yeast or lactic acid bacterium for fermentation is $1 \times 10^4 \sim 1 \times 10^5$ cells per 1 L of total culture solution. According to each strain, conventional culture conditions, aerobic or facultatively anaerobic conditions can be used. To activate the culture of a fermentative organism, additional carbon source and/or pH regulator can be added. Preferable culture conditions are as follows; 30 ~ 37 °C, pH 5 ~ 7, aerobic or facultatively anaerobic condition, for 1 ~ 15 days, preferably 1 ~ 7 days and more preferably 1 ~ 5 days and most preferably 2 ~ 4 days.

In step c), the fermented liquor obtained in step b) is primarily filtered. The filtrate is treated with protease in this step. At this time, the fermented liquor is filtered by 0.25 ~ 0.45 $\mu$m filter membrane to eliminate remaining *Phaseolus radiatus* powder particles and fermentative organism. The filtrate primarily filtered using the filter membrane is treated with protease to secondarily proteolysis or transform those ingredients not degraded or transformed by the fermentation using yeast or lactic acid bacterium. The protease used at this time is pepsin, trypsin, carboxypeptidase, aminopeptidase, dipeptidase, etc, and preferably pepsin, trypsin, carboxypeptidase, etc, and more preferably pepsin. In step d), the extract obtained by bio-transformation using protease is cold-aged, leading to stabilization. Secondary filtering is performed using a finer filter membrane, followed by concentration and stored. The concentrate is properly diluted for use. The enzyme treatment-filtering-concentration processes are the steps to prepare more effective *Phaseolus radiatus* fermentation-enzyme extract. At this time, cold-aging is preferably performed for 5 ~ 10 days and filter-size of the filter membrane used herein is preferably up to 0.25 $\mu$m.

In a preferred embodiment of the present invention, *Phaseolus radiatus* was grinded into 300 mesh sized or minuter powder, which was added to culture solution (10 g/L). 50,000 cfu/L of yeast or lactic acid bacterium was added thereto. Yeast or lactic acid bacterium was cultured for 1 ~ 7 days in a fermentor under the condition of 30 °C (yeast) or 37 °C (lactic acid bacterium); pH 5 ~ 7, preferably 5.5; aerobic or facultatively anaerobic condition, respectively. Upon completion of the culture, the fermented liquor was primarily filtered with 0.45 $\mu$m filter membrane. The filtrate was treated with protease. The protease treated solution was cold-aged for 10 days, followed by secondary filtering with 0.25 $\mu$m filter membrane. The filtrate was concentrated to give the *Phaseolus radiatus* extract offermentation-enzyme treatment of the present invention. The concentration of final solid powder was maintained as 10g/L before use.

The *Phaseolus radiatus* extract offermentation-enzyme treatment prepared by the processes described above demonstrated increased content of isoflavone (Experimental Example 1), increased collagen biosynthesis (Experimental Example 2), improved cellular activity (Experimental Example 3) and alleviated skin cell irritation (Experimental Example 4). So, a cosmetic composition containing the extract has the effect of improving wrinkles (Experimental Example 5) and skin moisturizing (Experimental Example 6). Therefore, it is expected that the *Phaseolus radiatus* fermentation-enzyme extract can be developed as a cosmetic composition effective in preventing skin aging.

In this invention, the content of *Phaseolus radiatus* fermentation-enzyme extract in the total cosmetic composition is 0.0001 ~ 30.0% (v/v) and more preferably 0.001 ~ 20% (v/v). If the content of *Phaseolus radiatus* extract offermentation-enzyme treatment is less than 0.0001% (v/v), the effect of improving wrinkles and skin protection cannot be expected. If the content of *Phaseolus radiatus* extract of fermentation-enzyme treatment is more than 30.0% (v/v), the effect is not significant even though the content is increased, and rather skin irritation occurs or stability of formulation is in question.

The effect of the *Phaseolus radiatus* extract offermentation-enzyme treatment of the present invention can be achieved by applying or spraying it locally on skin. In a preferred embodiment of the present invention, the composition of the present invention can be formulated as a cosmetic composition such as cream, lotion or emulsion or a pharmaceutical composition such as spray or ointment.

In addition to the *Phaseolus radiatus* extract offermentation-enzyme treatment, the active ingredient, the cosmetic composition of the present invention can additionally include a supplement generally used in the field of skin science such as antioxidants, stabilizers, solubilizer, vitamins, dyes, colorant or fragrant, and carriers.

The cosmetic composition of the present invention can be prepared in any type of formulation generally accepted in this field, which is exemplified by solution, suspension, emulsion, paste, gel, cream, lotion, powder, soap, surfactant-containing cleansing, oil, powdered foundation, emulsified foundation, waxed foundation and spray, but not always limited thereto. More particularly, the composition can be prepared as soft lotion (skin), nutrition lotion (milk lotion), nutrition cream, massage cream, essence, eye cream, cleansing cream, cleansing foam, cleansing water, pack, spray or powder.

In the case that the cosmetic composition is formulated as paste, cream or gel, the proper carrier can be selected from the group consisting of animal oil, vegetable oil, wax, paraffin, starch, tragacanth, cellulose derivative, polyethylene glycol, silicon, bentonite, silica, talc and zinc oxide.

In the case that the cosmetic composition is formulated as powder or spray, the proper carrier can be selected from the group consisting of lactose, talc, silica, aluminium hydroxide, calcium silicate and polyamide powder, and in particular if the composition of the present invention is formulated as spray, a propellant such as chlorofluorohydrocarbon, propane/butane or dimethyl ether can be additionally included.

In the case that the cosmetic composition is formulated as solution or emulsion, the proper carrier can be selected from the group consisting of solvent, solubilizer and emulsifier, which is exemplified by water, ethanol, isopropanol, ethyl carbonate, ethyl acetate, benzyl alcohol, benzyl benzoate, propylene glycol, 1,3-butyl glycol oil, glycerol aliphatic ester, polyethylene glycol and fatty acid ester of sorbitan.

In the case that the cosmetic composition is formulated as suspension, the proper carrier can be selected from the group consisting of liquid diluent such as water, ethanol or propylene glycol; suspending agent such as ethoxylated isostearyl alcohol, polyoxyethylene sorbitol ester and polyoxyethylene sorbitan ester; microcrystalline cellulose; aluminum methahydroxide; bentonite; agar; and tragacanth.

In the case that the cosmetic composition is formulated as surfactant-containing cleansing, the proper carrier can be selected from the group consisting of aliphatic alcohol sulfate, aliphatic alcohol ether sulfate, sulfosuccinic monoester, isethionate, imidazolinum derivative, methyltaurate, sarcosinate, fatty acid amide ether sulfate, alkyl amidobetain, aliphatic alcohol, fatty acid glyceride, fatty acid diethanolamide, vegetable oil, lanolin derivative and ethoxylated glycerol fatty acid ester.

The present invention also provides a make-up method characterized by applying the cosmetic composition containing *Phaseolus radiatus* extract offermentation-enzyme treatment on human skin so as to improve various skin troubles caused by aging (ex. increase cellular activity and collagen biosynthesis, alleviate skin cell irritation, moisturize skin, etc).

The make-up method of the present invention indicates every method of putting on cosmetics containing the step of applying a cosmetic composition on human skin. That is, every method of applying the cosmetic composition on skin, known to those in the art, is included in the criteria of the present invention.

The cosmetic composition of the present invention can be applied once or multiple times or co-administered with other cosmetic compositions. The cosmetic composition having excellent skin protective effect can be used according to the conventional method, and administration frequency varies with the individual skin condition or preference.

If the cosmetic composition of the present invention is formulated as soap, surfactant containing cleansing or surfactant non-containing cleansing, it is first applied on skin and then wiped out or washed out with water. At this time, the soap can be liquid soap, powdered soap, solid soap or oil type soap, and the surfactant containing cleansing formulation is exemplified by cleansing foam, cleansing water, cleansing towel and cleansing pack. The surfactant non-containing cleansing formulation is exemplified by cleansing cream, cleansing lotion, cleansing water and cleansing gel, but not always limited thereto.

The make-up method of the present invention to apply the cosmetic composition containing *Phaseolus radiatus* extract offermentation-enzyme treatment on human skin brings the effects of skin aging prevention, wrinkle improvement and skin moisturizing.

## Mode for the Invention

The advantages, features and aspects of the invention will become apparent from the following description of examples. It is to be understood, however, that these examples are illustrative only, and the scope of the present invention is not limited thereto.

### Comparative Manufacturing Example 1. Preparation of *Phaseolus radiatus* extract

*Phaseolus radiatus* was washed with purified water and then dried, followed by grinding. 100 g of the powder was added to 0.6 L of 70 % ethanol aqueous solution, followed by heat-extraction for 4 hours using reflux extractor equipped with cooler condenser. Then, the obtained extract was filtered with 300 mesh filter cloth and filtered again with Whatman #5 filter paper. The filtrate was concentrated by using the rotary vacuum evaporator to remove the solvent therefrom, and freeze-dried. As a result, 5.5 g (dried weight) of extract was obtained. The extract was named ' *Phaseolus radiatus* extract' and the final concentration was adjusted to 1 g/100 mL for use.

**Comparative Manufacturing Example 2. Preparation of *Phaseolus radiatus* liquor of yeast-fermentation**

*Phaseolus radiatus* was washed with purified water and dried, followed by grinding and filtering with 300 mesh filter cloth to produce fine powder. *Phaseolus radiatus* powder and purified water were mixed at the ratio of 1:10, which was sterilized at 121 °C. The mixture was inoculated with yeast. The yeast used herein was *Saccharomyces cerevisiae* and the concentration was 50,000 cfu/L. Culture was performed in a 5 L fermentor for 3 days at 30 °C under pH 5.5. Upon completion of the culture, the culture solution was centrifuged to remove the yeast primarily. The culture solution was filtered with 0.25 $\mu$m filter membrane. The filtrate was concentrated. The concentrate was diluted with a proper solvent. The prepared fermented liquor was named ' P *haseolus* radiatus liquor of yeast-fermentation' and the final concentration was adjusted to 1 g/100 mL for use.

**Comparative Manufacturing Example 3. Preparation of *Phaseolus radiatus* liquor of lactic acid bacterium-fermentation using *Lactobacillus***

*Phaseolus radiatus* was washed with purified water and dried, followed by grinding and filtering with 300 mesh filter cloth to produce fine powder. *Phaseolus radiatus* powder and purified water were mixed at the ratio of 1:10, which was sterilized at 121 °C. The mixture was inoculated with lactic acid bacterium. The lactic acid bacterium used herein was *Lactobacillus* spand the concentration was 50,000 cfu/L. Culture was performed in a 5 L fermentor for 3 days at 37 °C under pH 5.5. Upon completion of the culture, the culture solution was centrifuged to remove the bacterium primarily. The culture solution was filtered with 0.25 $\mu$m filter membrane. The filtrate was concentrated. The concentrate was diluted with a proper solvent. The prepared fermented liquor was named ' *Phaseolus radiatus* liquor oflactic acid bacterium-fermentation' and the final concentration was adjusted to 1 g/100 mL for use.

**Comparative Manufacturing Example 4. Preparation of *Phaseolus radiatus* extract of enzyme treatment-yeast fermentation**

*Phaseolus radiatus* was washed with purified water and dried, followed by grinding and filtering with 300 mesh filter cloth to produce fine powder. *Phaseolus radiatus* powder and purified water were mixed at the ratio of 1:10, which was sterilized at 121 °C. The mixture was treated with protease (pepsin, Sigma, USA) for 2 hours and inoculated with yeast. The yeast used herein was *Saccharomyces cerevisiae* and the concentration was 50,000 cfu/L. Culture was performed in a 5 L fermentor for 3 days at 30 °C under pH 5.5. Upon completion of the culture, the culture solution was centrifuged, followed by primary filtering to eliminate the yeast and debris. Then, secondary filtration was performed with 0.25 $\mu$m filter membrane. The filtrate was then concentrated and diluted with a proper solvent. The prepared fermented liquor was named ' *Phaseolus radiatus* extract of enzyme treatment-yeast fermentation' and the final concentration was adjusted to 1 g/100 mL for use.

**Comparative Manufacturing Example 5. Preparation of *Phaseolus radiatus* extract of enzyme treatment-lactic acid bacterium fermentation**

*Phaseolus radiatus* was washed with purified water and dried, followed by grinding and filtering with 300 mesh filter cloth to produce fine powder. *Phaseolus radiatus* powder and purified water were mixed at the ratio of 1:10, which was sterilized at 121 °C. The mixture was treated with protease (pepsin, Sigma, USA) for 2 hours and inoculated with lactic acid bacterium. The lactic acid bacterium used herein was *Lactobacillus* spand the concentration was 50,000 cfu/L. Culture was performed in a 5 L fermentor for 3 days at 37 °C under pH 5.5. Upon completion of the culture, the culture solution was centrifuged, followed by primary filtering to eliminate the bacterium and debris. Then, secondary filtration was performed with 0.25 $\mu$m filter membrane. The filtrate was then concentrated and diluted with a proper solvent. The prepared fermented liquor was named ' P*haseolus radiatus* extract of enzyme treatment-lactic acid bacterium fermentation' and the final concentration was adjusted to 1 g/100 mL for use.

**Manufacturing Example 1. Preparation of *Phaseolus radiatus* extract of yeast fermentation-enzyme treatment**

*Phaseolus radiatus* was washed with purified water and dried, followed by grinding and filtering with 300 mesh filter cloth to produce fine powder. *Phaseolus radiatus* powder and purified water were mixed at the ratio of 1:10, which was sterilized at 121 °C. The mixture was inoculated with yeast. The yeast used herein was *Saccharomyces cerevisiae* and the concentration was 50,000 cfu/L. Culture was performed in a 5 L fermentor for 3 days at 30 °C under pH 5.5.

Upon completion of the culture, the culture solution was centrifuged to remove the yeast, followed by primary filtering with 0.45 $\mu$m filter membrane. The filtrate was treated with protease (pepsin, Sigma, USA) for 2 hours, followed by secondary filtering with 0.25 $\mu$m filter membrane. The filtrate was then concentrated and diluted with a proper solvent. The prepared fermentation-enzyme extract was named ' *Phaseolus radiatus* extract of yeast fermentation-enzyme treatment' and the final concentration was adjusted to 1 g/100 mL for use.

**Manufacturing Example 2. Preparation of *Phaseolus radiatus* extract of lactic acid bacterium fermentation-enzyme treatment**

*Phaseolus radiatus* was washed with purified water and dried, followed by grinding and filtering with 300 mesh filter cloth to produce fine powder. *Phaseolus radiatus* powder and purified water were mixed at the ratio of 1:10, which was sterilized at 121 °C. The mixture was inoculated with lactic acid bacterium. The lactic acid bacterium used herein was *Lactobacillus* spand the concentration was 50,000 cfu/L. Culture was performed in a 5 L fermentor for 3 days at 30 °C under pH 5.5. Upon completion of the culture, the culture solution was centrifuged to remove the bacterium, followed by primary filtering with 0.45 $\mu$m filter membrane. The filtrate was treated with protease (pepsin, Sigma, USA) for 2 hours, followed by secondary filtering with 0.25 $\mu$m filter membrane. The filtrate was then concentrated and diluted with a proper solvent. The prepared fermentation-enzyme extract was named ' *Phaseolus radiatus* extract of lactic acid bacterium fermentation-enzyme treatment'and the final concentration was adjusted to 1 g/100 mL for use.

**Experimental Example 1: Analysis of isoflavone content in *Phaseolus radiatus* extract of fermentation-enzyme treatment**

To measure isoflavone content, the samples prepared in Manufacturing Examples were analyzed by high performance liquid chromatography (HPLC). And the results are shown in Table 1.

Table 1

[Table 1]

[Table]

| Comparison of isoflavone content | | | | | | | |
|---|---|---|---|---|---|---|---|
| Isoflavone | Comparative Manufacturing Example 1 | Comparative Manufacturing Example 2 | Comparative Manufacturing Example 3 | Comparative Manufacturing Example 4 | Comparative Manufacturing Example 5 | Manufacturing Example 1 | Manufacturing Example 2 |
| Daidzin | 450 | 610 | 630 | 720 | 730 | 1,040 | 1,050 |
| Glycitin | 410 | 580 | 590 | 680 | 670 | 1,040 | 1,040 |
| Genistin | 0 | 100 | 110 | 220 | 210 | 1,020 | 1,010 |
| Total | 960 | 1,290 | 1,330 | 1,620 | 1,610 | 3,100 | 3,090 |

The results indicate that isoflavone content was significantly increased in the samples of Manufacturing Example 1 and Manufacturing Example 2, which were treated with enzyme after fermentation, compared with other samples.

**Experimental Example 2: Increasing effect of *Phaseolus radiatus* extract of fermentation-enzyme treatment on collagen biosynthesis**

Human normal fibroblasts were inoculated in a 48-well microplate ($1 \times 10^6$ cells/ well) containing DMEM (Dulbecco's Modified Eagle Medium), followed by culture at 37 °C for 24 hours. The *Phaseolus radiatus* extract prepared in Comparative Manufacturing Example 1, the *Phaseolus radiatus* liquor of yeast-fermentation in Comparative Manufacturing Example 2, the *Phaseolus radiatus* liquor of lactic acid bacterium-fermentation prepared in Comparative Manufacturing Example 3, the *Phaseolus radiatus* extract of enzyme treatment-yeast fermentation prepared in Comparative Manufacturing Example 4, the *Phaseolus radiatus* extract of enzyme treatment-lactic acid bacterium fermentation prepared in Comparative Manufacturing Example 5, the *Phaseolus radiatus* extract of yeast fermentation-enzyme treatment prepared in Manufacturing Example 1 and the *Phaseolus radiatus* extract of lactic acid bacterium fermentation-enzyme treatment prepared in Manufacturing Example 2 were treated to the experimental group in which the final concentration of the respective extracts or liquors was 1.0 % and medium was replaced with serum-free DMEM medium, followed by further culture for 24 hours. What neither extracts nor liquors was contained was treated to the control in which medium was replaced with serum-free DMEM medium, followed by further culture for 24 hours. After the culture, supernatant of each well was collected and procollagen type I C-peptide (PICP) level therein was measured using the kit (Takara, Japan) to calculate the amount of collagen newly synthesized. PICP level was calculated as ng/mℓ. 250 uM of ascorbic acid was used as the positive control. The increasing rate of collagen biosynthesis was calculated according to the following Math Figure 1 and the results are shown in Table 2.
MathFigure 1

[Math.1]

$$\text{Increasing rate of collagen biosynthesis}(\%) = \left(\frac{\text{collagen amount of experimental group}}{\text{collagen amount of control}} - 1\right) \times 100$$

Table 2
[Table 2]

[Table]

| Increasing effect on collagen biosynthesis | |
|---|---|
| | Increasing rate of collagen biosynthesis (%) |
| Positive control (Ascorbic acid 250 uM) | 72.8 |
| Comparative Manufacturing Example 1 | 28.6 |
| Comparative Manufacturing Example 2 | 35.5 |
| Comparative Manufacturing Example 3 | 36.8 |
| Comparative Manufacturing Example 4 | 56.4 |
| Comparative Manufacturing Example 5 | 55.8 |
| Manufacturing Example 1 | **96.8** |
| Manufacturing Example 2 | **98.6** |

From the results of Experimental Example 2, it was confirmed that the *Phaseolus radiatus* extract of fermentation-enzyme treatment of Manufacturing Examples 1 and 2 of the present invention demonstrated more excellent increasing effect on collagen biosynthesis than the *Phaseolus radiatus* extract of Comparative Manufacturing Example 1, the simple fermented liquors (the *Phaseolus radiatus* liquor of yeast-fermentation of Comparative Manufacturing Example 2 and the *Phaseolus radiatus* liquor of lactic acid-fermentation of Comparative Manufacturing Example 2) and those *Phaseolus radiatus* extract of enzyme treatment-fermentation obtained from fermentation of *Phaseolus radiatus* extract after enzyme treatment (Comparative Manufacturing Examples 4 and 5). In addition, the *Phaseolus radiatus* extract of fermentation-enzyme treatment of the present invention significantly increased collagen biosynthesis, compared with the positive control ascorbic acid.

**Experimental Example 3: Increasing effect of *Phaseolus radiatus* extract of fermentation-enzyme treatment on fibroblast proliferation**

Human normal fibroblasts were inoculated in a 96-well microplate ($1 \times 10^4$ cells/ well) containing DMEM, followed by culture at 37 °C for 24 hours. The *Phaseolus radiatus* extract prepared in Comparative Manufacturing Example 1, the *Phaseolus radiatus* liquor of yeast-fermentation prepared in Comparative Manufacturing Example 2, the *Phaseolus radiatus* liquor of lactic acid bacterium-fermentation prepared in Comparative Manufacturing Example 3, the *Phaseolus radiatus* extract of enzyme treatment-yeast fermentation prepared in Comparative Manufacturing Example 4, the *Phaseolus radiatus* extract of enzyme treatment-lactic acid bacterium fermentation prepared in Comparative Manufacturing Example 5, the *Phaseolus radiatus* extract of yeast fermentation-enzyme treatment prepared in Manufacturing Example 1 and the *Phaseolus radiatus* extract of lactic acid bacterium fermentation-enzyme treatment prepared in Manufacturing Example 2 were treated to the experimental group in which the final concentration of the respective extracts or liquors was 1 % and medium was replaced with serum-free medium, followed by further culture for 24 hours. What neither extracts nor liquors was contained was treated to the control in which medium was replaced with serum-free DMEM medium, followed by further cultured for 24 hours, too. Upon completion of the culture, cell survival rate was compared. Particularly, MTT (Sigma, USA) solution (3 mg/ml) was added thereto and then Absorbance at 570 nm was measured using ELISA reader (Molecular Devices, USA). Proliferation rate (%) of fibroblast was calculated by the following Math Figure 2 and the results are shown in Table 3. TGF-β was used as the positive control.
MathFigure 2

[Math.2]

$$\text{Proliferation rate of fibroblast (\%)} = \left(\frac{\text{Absorbance of experimental group} - \text{Absorbance of control}}{\text{Absorbance of control}}\right) \times 100$$

Table 3
[Table 3]

[Table]

| Increasing effect on fibroblast proliferation | |
|---|---|
| | Proliferation rate of fibroblast (%) |
| Positive control (TGF-β 10ng/mℓ) | 186.6 |
| Comparative Manufacturing Example 1 | 120.8 |
| Comparative Manufacturing Example 2 | 133.4 |
| Comparative Manufacturing Example 3 | 138.6 |
| Comparative Manufacturing Example 4 | 148.6 |
| Comparative Manufacturing Example 5 | 142.6 |
| Manufacturing Example 1 | **182.6** |
| Manufacturing Example 2 | **188.6** |

As shown in Table 3, the *Phaseolus radiatus* extract of fermentation-enzyme treatment of Manufacturing Examples 1 and 2 of the present invention demonstrated more excellent increasing effect on cell proliferation than the *Phaseolus radiatus* extract of Comparative Manufacturing Example 1, the simple fermented liquors (the *Phaseolus radiatus* liquor of yeast-fermentation of Comparative Manufacturing Example 2 and the *Phaseolus radiatus* liquor of lactic acid-fermentation of Comparative Manufacturing Example 3) and those *Phaseolus radiatus* extract of enzyme treatment-fermentation obtained from fermentation of *Phaseolus radiatus* extract after enzyme treatment (Comparative Manufacturing Examples 4 and 5). The increasing effect of the *Phaseolus radiatus* extract of fermentation-enzyme treatment of the present invention on cell proliferation was as high as that of TGF-β, the positive control.

**Experimental Example 4: Alleviating effect of *Phaseolus radiatus* extract of fermentation-enzyme treatment on cell irritation caused by lactic acid**

Human skin cells, fibroblasts (KCTC, Korean Collection for Type Culture, Korea), were cultured in T-75 flask (Falcon, USA) until the cell confluency reached 80 %. The cells were transferred to a 96-well plate (Falcon, USA) at the density of $3 \times 10^4$ cells/ well, followed by further culture for 24 hours. After the culture, the cells were observed under microscope if they were completely adhered and well-grown. As a stimulus for skin, lactic acid (0.2 %) was used. Particularly, 200 $\mu\ell$ of each DMEM supplemented with 0.2 % lactic acid and DMEM not-containing lactic acid was added to each well, to which the *Phaseolus radiatus* extract, the *Phaseolus radiatus* liquor of yeast-fermentation, the *Phaseolus* radiatus liquor of lactic acid bacterium-fermentation, the *Phaseolus radiatus* extract of enzyme treatment-yeast fermentation, the *Phaseolus radiatus* extract of enzyme treatment-lactic acid bacterium fermentation (Comparative Manufacturing Examples 1-5), and the *Phaseolus radiatus* extract of fermentation-enzyme treatment (Manufacturing Examples 1 and 2) were added thereto at the concentration of 1.0 % (v/v). At that time, the control group was treated with neither lactic acid, *Phaseolus radiatus* extract, *Phaseolus radiatus* liquor of fermentation, *Phaseolus radiatus* extract of enzyme treatment-fermentationnor *Phaseolus radiatus extract of* fermentation-enzyme treatment. The comparative group was only treated with lactic acid. After 12 hours from the treatment with test samples, cell survival rate was compared. Particularly, MTT (Sigma, USA) solution (3 mg/ml) was added thereto and then Absorbance at 570 nm was measured using ELISA reader (Molecular Devices, USA). Cell survival rate (%) was calculated by the following Math Figure 3 and the results are shown in Table 4.

MathFigure 3

[Math.3]

$$Cell\ survival\ rate\ (\%) = \left(\frac{Absorbance\ of\ control - Absorbance\ of\ test\ sample}{Absorbance\ of\ control}\right) \times 100$$

Table 4

[Table 4]

[Table]

| Alleviating effect of *Phaseolus radiatus* fermentation-enzyme extract on cell irritation | |
|---|---|
| | Proliferation rate of fibroblast (%) |
| Control | 98.8 |
| Comparative control (lactic acid 0.2%) | 0.0 |
| Comparative Manufacturing Example 1 (1%) | 98.6 |
| Comparative Manufacturing Example 1 (1%)+ lactic acid 0.2% | 56.4 |
| Comparative Manufacturing Example 2 (1%) | 100.2 |
| Comparative Manufacturing Example 2 (1%)+ lactic acid 0.2% | 60.2 |
| Comparative Manufacturing Example 3 (1%) | 100.0 |
| Comparative Manufacturing Example 3 (1%)+ lactic acid 0.2% | 60.4 |
| Comparative Manufacturing Example 4 (1%) | 98.8 |
| Comparative Manufacturing Example 4 (1%)+ lactic acid 0.2% | 66.8 |
| Comparative Manufacturing Example 5 (1%) | 98.8 |
| Comparative Manufacturing Example 5 (1%)+ lactic acid 0.2% | 64.8 |
| Manufacturing Example 1 (1%) | 98.8 |
| Manufacturing Example 1 (1%) + lactic acid 0.2% | 72.6 |
| Manufacturing Example 2 (1%) | 100.0 |
| Manufacturing Example 2 (1%) + lactic acid 0.2% | 74.6 |

As shown in Table 4, the *Phaseolus radiatus* extract of fermentation-enzyme treatment prepared in Manufacturing Examples 1 and 2 of the present invention demonstrated more excellent increasing effect on cell survival rate than the *Phaseolus radiatus* extract of Comparative Manufacturing Example 1, suggesting that they had excellent alleviating effect of cytotoxicity caused by the treatment of lactic acid, and demonstrated more excellent increasing effect on cell survival rate than the simple *Phaseolus radiatus* liquor of fermentation (the *Phaseolus radiatus* liquor of yeast-fermentation of Comparative Manufacturing Example 2 and the *Phaseolus radiatus* liquor of lactic acid-fermentation of Comparative Manufacturing Example 3) and the *Phaseolus radiatus* extract of enzyme treatment-fermentation obtained from fermentation of *Phaseolus radiatus* extract after enzyme treatment (Comparative Manufacturing Examples 4 and 5), suggesting that they had excellent cell stimulus alleviation effect.

Based on the above results, the cosmetic composition containing the *Phaseolus radiatus* extract of fermentation-enzyme treatment of the present invention is provided as follows. The composition of the present invention is not limited to the following examples, though. The *Phaseolus radiatus* extract of fermentation-enzyme treatment included in the composition can be either *Phaseolus radiatus* extract of yeast fermentation-enzyme treatment or *Phaseolus radiatus* extract of lactic acid bacterium fermentation-enzyme treatment.

**Formulation 1: Soft lotion**

Soft lotion containing *Phaseolus radiatus* extract of fermentation-enzyme treatment was prepared based on the constitutions as follows.

Table 5

[Table 5]

[Table]

| Constituent | Content (unit: weight %) |
|---|---|
| *Phaseolus radiatus* extract of fermentation-enzyme treatment | 0.5 |
| Glycerine | 5.0 |
| 1.3-butyleneglycol | 3.0 |
| Allantoin | 0.1 |
| DL-panthenol | 0.3 |
| E.D.T.A-2NA | 0.02 |
| Benzophenone-9 | 0.04 |
| Sodium hyaluronate | 5.0 |
| Nicol HCO 60 | 0.4 |
| Methyl paraben | 0.2 |
| Fragrant | 0.01 |
| Distilled water | remainder |
| Total | 100 |

**Formulation 2: Nutrition lotion**

Nutrition lotion containing *Phaseolus radiatus* extract of fermentation-enzyme treatment was prepared based on the constitutions as follows.

Table 6

[Table 6]

[Table]

| Constituent | Content (unit: weight%) |
|---|---|
| *Phaseolus radiatus* extract of fermentation-enzyme treatment | 2.0 |
| Glyceryl stearate SE | 1.5 |

(continued)

| Constituent | Content (unit: weight%) |
|---|---|
| Stearyl alcohol | 1.5 |
| Lanolin | 1.5 |
| Polysorbate 60 | 1.3 |
| Sorbitan stearate | 0.5 |
| Hydrogenated vegetable oil | 1.0 |
| Mineral oil | 5.0 |
| Squalane | 3.0 |
| Trioctanoin | 2.0 |
| Dimethicone | 0.8 |
| Tocopherol acetate | 0.5 |
| Carboxyvinylpolymer | 0.12 |
| Glycerine | 5.0 |
| 1.3-butyleneglycol | 3.0 |
| Sodium hyaluronate | 5.0 |
| Triethanolamine | 0.12 |
| Methyl paraben | 0.2 |
| Fragrant | 0.02 |
| Distilled water | remainder |
| Total | 100 |

**Formulation 3: Nutrition cream**

Nutrition cream containing *Phaseolus radiatus* extract of fermentation-enzyme treatment was prepared based on the constitutions as follows.
Table 7
[Table 7]

[Table]

| Constituent | Content (unit: weight%) |
|---|---|
| *Phaseolus radiatus* extract of fermentation-enzyme treatment | 3.0 |
| Glyceryl monostearate, Lipophilic | 2.0 |
| Cetearyl alcohol | 2.2 |
| Stearic acid | 1.5 |
| Wax | 1.0 |
| Polysorbate 60 | 1.5 |
| Sorbitan stearate | 0.6 |
| Hydrogenated vegetable oil | 1.0 |
| Squalane | 3.0 |
| Mineral oil | 5.0 |
| Trioctanoin | 5.0 |

(continued)

| Constituent | Content (unit: weight%) |
|---|---|
| Dimethicone | 1.0 |
| Sodium magnesium silicate | 0.1 |
| Glycerine | 5.0 |
| Betain | 3.0 |
| Triethanolamine | 1.0 |
| Sodium hyaluronate | 4.0 |
| Methyl paraben | 0.2 |
| Fragrant | 0.05 |
| Distilled water | remainder |
| Total | 100 |

**Experimental Example 5: Evaluation of wrinkle improvement effect of cosmetics containing *Phaseolus radiatus* extract of fermentation-enzyme treatment**

Clinical test was performed to investigate wrinkle improvement effect of the cosmetic composition of the present invention. The nutrition cream of Formulation 3 containing 3 % (v/v) of the *Phaseolus radiatus* extract of lactic acid bacterium fermentation-enzyme treatment of Manufacturing Example 2, the comparative cream which was manufactured by the identical method with the manufacturing method of Formulation 3 except for using the *Phaseolus radiatus* extract of enzyme treatment-lactic acid bacterium-fermentation of Comparative Manufacturing Example 5 instead of the *Phaseolus radiatus* extract of lactic acid bacterium fermentation-enzyme treatment of Manufacturing Example 2 (Comparative Formulation), and the control cream which was manufactured by the identical method with the manufacturing method of Formulation 3 except for using purified water instead of the *Phaseolus radiatus* extract of lactic acid bacterium fermentation-enzyme treatment of Manufacturing Example 2 (Control Formulation) were used for this test. Test samples were applied on faces of 90 women, on both chicks, for 6 weeks. Wrinkles were observed 6 weeks later by the naked eye to evaluate wrinkle improvement effect. And the results are shown in Table 8.
Table 8
[Table 8]

[Table]

| Wrinkle improvement effect of nutrition cream containing *Phaseolus radiatus* extract of fermentation-enzyme treatment | | | | |
|---|---|---|---|---|
| Cosmetics | Wrinkle improvement effect | | | Efficiency (%) |
| | Good | Moderate | No | |
| Cream of Formulation 3 | 20 | 5 | 5 | 83.3 |
| Cream of Comparative Formulation | 18 | 2 | 10 | 66.7 |
| Cream of Control Formulation | 3 | 3 | 24 | 20.0 |

As shown in Table 8, the cream containing *Phaseolus radiatus* extract of fermentation-enzyme treatment of Formulation 3 of the present invention demonstrated significant wrinkle improvement effect, compared with the cream of Comparative Formulation containing the *Phaseolus radiatus* extract of enzyme treatment-lactic acid bacterium fermentataionof Comparative Manufacturing Example 5 instead of the *Phaseolus radiatus* extract of fermentation-enzyme treatment of the present invention and the cream of Control Formulation containing purified water instead of the *Phaseolus radiatus* extract of fermentation-enzyme treatment of the present invention. Skin irritation was not observed in most of subjects administered with the cosmetic composition of the present invention.

**Experimental Example 6: Evaluation of moisturizing effect of cosmetics containing *Phaseolus radiatus* extract of fermentation-enzyme treatment**

Clinical test was performed to investigate moisturizing effect of the cosmetic composition of the present invention. The nutrition cream of Formulation 3 containing 3 % (v/v) of the *Phaseolus radiatus* extract of lactic acid bacterium fermentation-enzyme treatment of Manufacturing Example 2, the comparative cream which was manufactured by the identical method with the manufacturing method of Formulation 3 except for using the *Phaseolus radiatus* extract of enzyme treatment-lactic acid bacterium fermentation of Comparative Manufacturing Example 5 instead of the *Phaseolus radiatus* extract of lactic acid bacterium fermentation-enzyme treatment of Manufacturing Example 2 (Comparative Formulation), and the control cream which was manufactured by the identical method with the manufacturing method of Formulation 3 except for using purified water instead of the *Phaseolus radiatus* extract of lactic acid bacterium fermentation-enzyme treatment of Manufacturing Example 2 (Control Formulation) were used for this test. 30 women were participated in this test. Square area on the brachium was set for the application of the cosmetics. The cosmetic composition was applied thereon for 2 weeks, during which skin moisture level was measured by using skin corneometer (C+K courage, Germany). The number of subjects showing at least 10% improvement was shown and the improvement of skin moisturizing was calculated in Table 9.
Table 9
[Table 9]

[Table]

| Moisturizing effect of nutrition cream containing *Phaseolus radiatus* fermentation-enzyme extract | | | |
|---|---|---|---|
| Cosmetics | Moisturizing effect | | Efficiency (%) |
| | ≥ 10% | No effect | |
| Cream of Formulation 3 | 24 | 6 | 80.0 |
| Cream of Comparative Formulation | 21 | 9 | 70.0 |
| Cream of Control Formulation | 10 | 20 | 33.3 |

As shown in Table 9, the cream containing *Phaseolus radiatus* extract of fermentation-enzyme treatment of Formulation 3 of the present invention demonstrated significant skin moisturizing effect, compared with the cream of Comparative Formulation containing the *Phaseolus radiatus* extract of enzyme treatment-lactic acid bacterium fermentation of Comparative Manufacturing Example 5 instead of the *Phaseolus radiatus* extract of fermentation-enzyme treatment of the present invention and the cream of Control containing purified water instead of the *Phaseolus radiatus* extract of fermentation-enzyme treatment of the present invention.

## Claims

1. A cosmetic composition containing *Phaseolus radiatus* extract of fermentation-enzyme treatment as an active ingredient for preventing skin aging and/or for improving skin wrinkles and/or for skin moisturizing, wherein the *Phaseolus radiatus* extract of fermentation-enzyme treatment is obtained by the proceses of preparing fermented liquor by fermenting *Phaseolus radiatus* with yeast or lactic acid bacterium, and enzyme-treating the fermented liquor with protease.

2. The cosmetic composition according to claim 1, wherein the yeast is *Saccharomyces cerevisiae.*

3. The cosmetic composition according to claim 1, wherein the protease is selected from the group consisting of pepsin, trypsin, carboxypeptidase, aminopeptidase and dipeptidase.

4. The cosmetic composition according to claim 1, wherein the lactic acid bacterium is *Lactobacillus.*

5. The cosmetic composition according to claim 1, wherein the *Phaseolus radiatus* extract of fermentation-enzyme treatment is included in the cosmetic composition at the concentration of 0.0001-30.0% (w/w).

6. The cosmetic composition according to claim 1, wherein the cosmetic composition is formulated in the form selected from the group consisting of liquid, suspension, emulsion, paste, gel, cream, lotion, powder, soap, surfactant-containing cleansing, oil, powdered foundation, emulsified foundation, waxed foundation and spray.

7. A make-up method **characterized by** applying the cosmetic composition of claim 1 on human skin to improve skin aging and/or skin wrinkles and/or skin moisturizing.

## Patentansprüche

1. Kosmetische Zusammensetzung, umfassend *Phaseolus*-radiatus-Extrakt aus Fermentation-Enzymbehandlung als Wirkstoff zum Verhindern von Hautalterung und/oder zum Verbessern von Hautfalten und/oder zur Hautbefeuchtung, wobei der *Phaseolus-radiatus*-Extrakt aus Fermentation-Enzymbehandlung durch die Verfahren der Herstellung von fermentierter Flüssigkeit durch Fermentieren von *Phaseolus radiatus* mit Hefe oder Milchsäurebakterium und Enzymbehandlung der fermentierten Flüssigkeit mit Protease erhalten ist.

2. Kosmetische Zusammensetzung gemäß Anspruch 1, wobei die Hefe *Saccharomyces cerevisiae* ist.

3. Kosmetische Zusammensetzung gemäß Anspruch 1, wobei die Protease ausgewählt ist aus der Gruppe bestehend aus Pepsin, Trypsin, Carboxypeptidase, Aminopeptidase und Dipeptidase.

4. Kosmetische Zusammensetzung gemäß Anspruch 1, wobei das Milchsäurebakterium *Lactobacillus* ist.

5. Kosmetische Zusammensetzung gemäß Anspruch 1, wobei der *Phaseolus-radiatus*-Extrakt aus Fermentation-Enzymbehandlung mit einer Konzentration von 0,0001-30,0 % (w/w) in der kosmetischen Zusammensetzung enthalten ist.

6. Kosmetische Zusammensetzung gemäß Anspruch 1, wobei die kosmetische Zusammensetzung in einer Form ausgewählt aus der Gruppe bestehend aus Flüssigkeit, Suspension, Emulsion, Paste, Gel, Creme, Lotion, Pulver, Seife, tensidhaltigem Reiniger, Öl, pulverförmiger Grundlage, emulgierter Grundlage, wachsartiger Grundlage und Spray formuliert ist.

7. Make-Up-Verfahren, **gekennzeichnet durch** Aufbringen der kosmetischen Zusammensetzung gemäß Anspruch 1 auf menschliche Haut zum Verbessern von Hautalterung und/oder Hautfalten und/oder Hautfeuchtigkeit.

## Revendications

1. Composition cosmétique contenant un extrait de *Phaseolus radiatus* issu d'un traitement par fermentation enzymatique, en tant qu'ingrédient actif pour prévenir le vieillissement de la peau et/ou pour améliorer les rides de la peau et/ou pour l'hydratation de la peau, dans laquelle l'extrait de *Phaseolus radiatus* issu du traitement par fermentation enzymatique est obtenu à l'aide du procédé de préparation de liqueur fermentée par fermentation du *Phaseolus radiatus* avec une levure ou une bactérie lactique, et traitement enzymatique de la liqueur fermentée par une protéase.

2. Composition cosmétique selon la revendication 1, dans laquelle la levure est *Saccharomyces cerevisiae.*

3. Composition cosmétique selon la revendication 1, dans laquelle la protéase est choisie dans le groupe constitué par la pepsine, la trypsine, la carboxypeptidase, l'aminopeptidase et la dipeptidase.

4. Composition cosmétique selon la revendication 1, dans laquelle la bactérie lactique est *Lactobacillus.*

5. Composition cosmétique selon la revendication 1, dans laquelle l'extrait de *Phaseolus radiatus* issu du traitement par fermentation enzymatique est incorporé dans la composition cosmétique à la concentration de 0,0001-30,0% (p/p).

6. Composition cosmétique selon la revendication 1, ladite composition cosmétique étant formulée sous la forme choisie dans le groupe constitué par le liquide, la suspension, l'émulsion, la pâte, le gel, la crème, la lotion, la poudre, le savon, le nettoyant contenant du tensioactif, l'huile, le fond de teint en poudre, le fond de teint émulsionné, le fond de teint à la cire et le spray.

7. Procédé de maquillage **caractérisé par** l'application de la composition cosmétique de la revendication 1 sur la peau humaine pour améliorer le vieillissement de la peau et/ou les rides de la peau et/ou l'hydratation de la peau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007007989 A
- KR 100753310